## Europäisches Patentamt
## European Patent Office
## Office européen des brevets

(11) Publication number: **0 018 177**
**B1**

(12) **EUROPEAN PATENT SPECIFICATION**

(45) Date of publication of patent specification: **24.03.82**

(21) Application number: **80301165.9**

(22) Date of filing: **11.04.80**

(51) Int. Cl.³: **C 07 C  99/10,**
**C 07 C  101/10,**
**C 07 C  121/43**

(54) Process for producing alpha-halogeno-beta-alanines or the mineral acid salts thereof.

(30) Priority: **12.04.79 JP 43643/79**
**18.04.79 JP 46635/79**
**03.08.79 JP 98634/79**
**14.08.79 JP 102736/79**

(43) Date of publication of application:
**29.10.80 Bulletin 80/22**

(45) Publication of the grant of the patent:
**24.03.82 Bulletin 82/12**

(84) Designated Contracting States:
**CH DE FR GB LI NL**

(56) References cited:
**GB - A - 848 611**

**CHEMICAL ABSTRACTS, vol. 62, nr. 11, May 24, 1965, ref. nr. 13234c**
**COLUMBUS OHIO (US)**

(73) Proprietor: **MITSUI TOATSU CHEMICALS, INCORPORATED**
**2-5 3-chome Kasumigaseki**
**Chiyoda-Ku Tokyo 100 (JP)**

(72) Inventor: **Mita, Ryuichi**
**529, Shimosakunobe Takatsu-ku**
**Kawasaki-shi Kanagawa-ken 213 (JP)**
Inventor: **Higuchi, Chojiro**
**4-5-13, Dai**
**Kamakura-shi Kanagawa-ken 247 (JP)**
Inventor: **Yamaguchi, Akihiro**
**1-1-21, Iwase**
**Kamakura-shi Kanagawa-ken 247 (JP)**
Inventor: **Murakami, Hisamichi**
**2-15-14, Fujizuka Kohoku-ku**
**Yokohama-shi Kanagawa-ken 222 (JP)**
Inventor: **Kato, Toshio**
**600-1, Kamisakunobe Takatsu-ku**
**Kawasaki-shi Kanagawa-ken 213 (JP)**

(74) Representative: **Armitage, Ian Michael et al,**
**MEWBURN ELLIS & CO. 2/3 Cursitor Street**
**London EC4A 1BQ (GB)**

Courier Press, Leamington Spa, England.

(58) References cited:

**CHEMISCHE BERICHTE, vol, 91, 1958
WEINHEIM (DE)
K. D. GUNDERMANN et al. "Äthylenimin-
carbonsäure-(2)-äthylester. DL-Serin und DL-
Isoserin aus $\alpha$-Chlor-$\beta$-amino-propionsäure",
pages 160—167.**

**HOUBEN-WEYL: "Methoden der organischen
Chemie, 4th Edition, 1952, Georg Thieme
Verlag, Stuttgart
Band VIII: "Sauerstoffverbindungen III" pages
429—432.**

**0 018 177**

### Process for producing alpha-halogeno-beta-alanines or the
### mineral acid salts thereof

This invention relates to a process for producing alpha-halogeno-beta-alanines or the mineral acid salts thereof which are useful as intermediates for the synthesis of alpha-amino acids such as serine or as intermediates for medicines.

Some methods for the production of alpha-halogeno-beta-alanines or the mineral acid salts thereof have previously been known. For example, there have been suggested (a) a method for producing alpha-chloro-beta-alanine hydrochloride which comprises reacting alpha-chloroacrylonitrile with ammonia water, treating the reaction mixture with benzoyl chloride, and hydrolyzing the resulting alpha-chloro-beta-benzoyl aminopropionitrile with hydrochloric acid (Japanese Patent Publication No. 30152/64), and (b) a method for producing alpha-chloro-beta-alanine hydrochloride which comprises reacting alpha-chloroacrylonitrile with phthalimide and hydrolyzing the resulting alpha-chloro-beta-[2-carbomethoxybenzoylamino]propionitrile with hydrochloric acid [K. D. Gundermann, Chem. Ber., Vol. 91, page 160 (1958)]. In both of these known methods, the hydrolysis is carried out while protecting the amino group with an acyl group, and it is necessary therefore to separate the desired alpha-chloro-beta-alanine hydrochloride from benzoic acid or phthalic acid formed as a by-product during hydrolysis. Moreover, the production of the intermediate, alpha-chloro-beta-benzoylaminopropionitrile or alpha-chloro-beta(2-carbomethoxybenzoylamino)propionitrile, poses problems in regard to the yield of the final product, the reaction operations, etc. Accordingly, these methods are not entirely satisfactory for commercial application.

It is an object of this invention therefore to provide a commercially advantageous process for producing alpha-halogeno-beta-alanines or the mineral acid salts thereof.

The present inventors made various investigations in order to achieve this object, and have found that alpha-halogeno-beta-alanines or the mineral acid salts thereof can be produced very easily in high yields by hydrolyzing alpha-halogeno-beta-aminopropionitrile mineral acid salts with mineral acids.

It has also been found in accordance with this invention that alpha-halogeno-beta-alanines or the mineral acid salts thereof can be produced in high yields by reacting an alpha-halogenoacrylonitrile or an alpha,beta-dihalogenopropionitrile with ammonia, isolating the resulting alpha-halogeno-beta-aminopropionitrile, and hydrolyzing it with a mineral acid.

It has further been found that alpha-halogeno-beta-alanines or the mineral acid salts thereof can be obtained in high yields by subjecting the mixture containing an alpha-halogeno-beta-aminopropionitrile obtained by reacting an alpha,beta-dihalogenopropionitrile with ammonia in water and/or an organic solvent to hydrolysis with a mineral acid without isolating the alpha-halogeno-beta-amino-propionitrile therefrom.

The present inventors have also found that alpha-halogeno-beta-alanines or the mineral acid salts thereof can be obtained in high yields by subjecting the reaction mixture containing an alpha-halogeno-beta-aminopropionitrile and obtained by the addition reaction of an alpha-halogenoacrylonitrile with ammonia to hydrolysis with a mineral acid without isolating the alpha-halogeno-beta-aminopropionitrile therefrom but optionally after removing the excess of ammonia.

Thus, according to this invention, there is provided a process for producing an alpha-halogeno-beta-alanine or a mineral acid salts thereof, which comprises hydrolyzing an alpha-halogeno-beta-aminopropionitrile or its mineral acid salts with a mineral acid.

As preferred embodiments of the aforesaid process, the present invention also provides a process which involves using as a starting material the reaction mixture containing an alpha-halogeno-beta-aminopropionitrile obtained by reacting an alpha,beta-dihalogenopropionitrile with ammonia in water and/or an organic solvent, and a process which involves using as a starting material the reaction mixture containing an alpha-halogeno-beta-aminopropionitrile obtained by the addition reaction of an alpha-halogenoacrylonitrile with ammonia.

The process of this invention for producing alpha-halogeno-beta-alanines or the mineral acid salts thereof by directly hydrolyzing alpha-halogeno-beta-aminopropionitrile or the mineral acid salts thereof is a novel process quite unknown in the past. This process has the advantage that the process steps can be markedly shortened, there is no necessity of separating the desired product from by-products, and the alpha-chloro-beta-alanines or the mineral acids thereof can be obtained in high yields. The process is therefore very advantageous in commercial application.

In the process of this invention, an alpha-halogeno-beta-aminopropionitrile or its mineral acid salts is used as a starting material. Examples of the starting material are alpha-chloro-beta-amino-propionitrile, alpha-bromo-beta-aminopropionitrile, and the mineral acid salts thereof, such as the hydrochlorides, sulfates, nitrates, and phosphates thereof. In commercial practice, alpha-chloro-beta-aminopropionitrile, or alpha-chloro-beta-aminopropionitrile hydrochloride or sulfate is frequently used.

The alpha-halogeno-beta-aminopropionitrile is usually obtained by reacting an alpha-halogenoacrylonitrile or an alpha,beta-dihalogenopropionitrile with ammonia in water and/or an organic solvent, and if desired, isolating the resulting product by extraction with a water-immiscible organic solvent, followed by distillation under reduced pressure, etc.

The alpha-halogeno-beta-aminopropionitrile mineral acid salt used, for example alpha-chloro-beta-aminopropionitrile hydrochloride, can be produced by reacting alpha-chloroacrylonitrile in a solution of ammonia gas in methanol, removing the excess of ammonia under reduced pressure, adding 25% methanolic hydrochloric acid at not more than —30°C, distilling off the methanol, and adding ether [L. Doub, J. Heterocycl. Chem., Vol. 7, page 532 (1970)]; or by reacting alpha-chloroacrylonitrile with ammonia in isopropanol, then removing the excess of ammonia, and then adding to the resulting solution a lower alcohol solution having dissolved therein hydrogen chloride in an amount slightly in excess of the stoichiometrical amount. Alpha-chloro-beta-aminopropionitrile sulfate can be produced, for example by reacting alpha-chloroacrylonitrile with ammonia in a lower alcohol such as isopropanol, removing the excess of ammonia, and adding to the resulting reaction solution an isopropanol solution having dissolved therein a slight excess of sulfuric acid. Other alpha-halogeno-beta-aminopropionitrile mineral acid salts can be produced by similar procedures. For use as the starting material in the process of this invention, these compounds need not to be of high purity, and may contain some amounts of by-products of the reactions.

The free alpha-halogeno-beta-aminopropionitrile can be obtained, for example, by reacting an alpha-halogenoacrylonitrile with ammonia water at low temperatures, extracting the product with a water-immiscible organic solvent, drying the extract, and then distilling it under reduced pressure. In the past, however, there has been no successful example of isolating free alpha-halogeno-beta-amino-propionitrile. Accordingly, the process of this invention for producing alpha-halogeno-beta-alanines or the mineral acid sats thereof from alpha-halogeno-beta-aminopropionitrile as a starting material has not been known heretofore.

In preferred embodiments of this invention, the alpha-halogeno-beta-alanines or the mineral acid salts thereof can be produced by (I) performing the addition reaction of an alpha-halogenoacrylonitrile with ammonia to form an alpha-halogeno-beta-aminopropionitrile in the reaction mixture, and subjecting the reaction mixture to hydrolysis with a mineral acid without isolating the alpha-halogeno-beta-aminopropionitrile therefrom; or (II) reacting an alpha,beta-dihalogenopropionitrile with ammonia to form an alpha-halogeno-beta-aminopropionitrile in the reaction mixture, and subjecting the reaction mixture to hydrolysis with a mineral acid without isolating the alpha-halogeno-beta-aminopropionitrile therefrom.

Examples of the alpha-halogenoacrylonitrile used in the embodiment (I) are alpha-chloroacrylonitrile and alpha-bromoacrylonitrile. Ammonia is used in the form of ammonia water or a solution of ammonia water or ammonia gas in a lower alcohol such as methanol, ethanol, n-propanol or isopropanol. When ammonia water is used, its ammonia concentration is suitably from 5 to 30% by weight. When ammonia gas is dissolved in a lower alcohol, the concentration of ammonia is selected within the range of 2 to 25% by weight. The amount of ammonia used is at least 1 mole, preferably at least 1.2 moles, per mole of the alpha-halogenoacrylonitrile.

The temperature of the addition reaction of the alpha-halogenoacrylonitrile with ammonia is from —40°C to 20°C, preferably from —20°C to 10°C. The reaction time is 2 to 10 hours, and usually a period of 3 to 6 hours is sufficient. Preferably, the reaction is allowed to proceed by gradually adding the alpha-halogenoacrylonitrile dropwise to ammonia water. By performing the reaction in a nitrogen atmosphere or a nitrogen stream, side-reactions such as the polymerization of the alpha-halogenoacrylonitrile can be inhibited. The end point of the reaction can be rapidly and simply determined, for example, by gas chromatography, high-speed liquid chromatography or thin-layer chromatography. Then, the resulting reaction mixture containing the alpha-halogeno-beta- aminopropionitrile is acidified with a mineral acid and then heated. As a result, the alpha-halogeno-beta-aminopropionitrile is hydrolyzed to form an alpha-halogeno-beta-alanine.

In the embodiment (II) described above, first the alpha,beta-dihalogenopropionitrile is reacted with ammonia in water and/or an organic solvent. Usually, alpha,beta-dichloropropionitrile or alpha,beta-dibromopropionitrile is frequently used as the alpha,beta-dihalogenopropionitrile in this reaction. These materials can be easily produced by chlorination or bromination of acrylonitrile. For commercial practice, alpha,beta-dichloropropionitrile is preferred. Ammonia is usually used in the form of ammonia water. Or it may be used in the form of a solution of ammonia gas or ammonia water in an organic solvent. Any organic solvents capable of dissolving ammonia can be used. Generally, lower alcohols such as methanol, ethanol, n-propanol, isopropanol, n-butanol, isobutanol, tertiary butanol, methyl Cellosolve (ethylene glycol monomethyl ether), or Cellosolve (ethylene glycol monoethyl ether), either alone or in combination with each other, are used. These organic solvents may be used as a mixture with water. The concentration of ammonia in water is 5 to 30% by weight, and the concentration of ammonia in the solution of ammonia gas or ammonia water in the lower alcohol is selected within the range of 2 to 25% by weight. The amount of ammonia used is at least 2 moles, preferably at least 2.2 moles, per mole of the alpha,beta-dihalogenopropionitrile. There is no particular restriction on the mode of the reaction. Usually, it is preferred to add gradually the alpha,beta-dihalo-genopropionitrile dropwise to ammonia water or to a solution of ammonia gas or ammonia water in a lower alcohol. By performing the reaction in a nitrogen atmosphere or a nitrogen stream, side-reactions such as the decomposition of the resulting alpha-halogeno-beta-aminopropionitrile can be markedly inhibited. In the reaction of the alpha,beta-dihalogenopropionitrile with ammonia, the reaction temperature is from —40°C to 30°C, preferably from —20°C to 20°C, and the reaction time is from 0.5 to 20 hours, preferably from 1 to 15 hours. The end point of the reaction can be rapidly and simply determined by such means as gas-chromatography, high-speed liquid chromatography or thin-layer chromatography. Subsequently, the resulting reaction mixture containing alpha-halogeno-beta-amino-propionitrile is acidified with a mineral acid and heated. As a result, the alpha-halogeno-beta-amino-propionitrile is hydrolyzed to form an alpha-halogeno-beta-alanine.

Examples of the mineral acid used for hydrolysis in the process of this invention are hydrochloric acid, sulfuric acid, nitric acid, and phosphoric acid. Hydrochloric acid and sulfuric acid are preferred. The concentration of the mineral acid used can be suitably selected within the range of 3 to 80% by weight. For example, the suitable concentration of hydrochloric acid is 3 to 35% by weight, preferably 8 to 30% by weight, and the suitable concentration of sulfuric acid is 3 to 60% by weight, preferably 15 to 55% by weight.

Accordingly, when the reaction mixture is to be acidified with a mineral acid after the addition reaction of the alpha-halogenoacrylonitrile with ammonia or the reaction of the alpha,beta-dihalogeno-propionitrile with ammonia, the concentration of the mineral acid in the reaction system is adjusted to the above-specified range in consideration of water and the excess ammonia remaining in the reaction system. The excess ammonia remaining in the reaction system may be removed in advance by such an operation as the blowing of nitrogen gas into the reaction system. This treatment will facilitate the adjustment of the concentration of the mineral acid.

The amount of the mineral acid used differs according to the concentration of the mineral acid. Usually, the concentration of the mineral acid is at least 2 parts by weight, preferably at least 3 parts by weight, per part by weight of the alpha-halogeno-beta-aminopropionitrile or its mineral acid salt. Such amounts are generally advantageous to reaction operations.

In subjecting the alpha-halogeno-beta-aminopropionitrile or its mineral acid salt as a starting material, the alpha-halogeno-beta-aminopropionitrile or its mineral acid salt is added to the mineral acid and the mixture is heated with stirring. When the alpha-halogeno-beta-aminopropionitrile-containing reaction mixture obtained by the addition reaction of the alpha-halogenoacrylonitrile with ammonia, or the alpha-halogeno-beta-aminopropionitrile-containing reaction mixture obtained by the reaction of the alpha,beta-dihalogenopropionitrile with ammonia is to be subsequently subjected to hydrolysis, the reaction mixture is acidified with a mineral acid by adding the reaction mixture to the mineral acid or adding the mineral acid to the reaction mixture, and the resulting mixture is heated with stirring. The hydrolysis temperature is 50 to 150°C, preferably 80 to 120°C, and the reaction time is 0.5 to 10 hours, generally 1 to 6 hours. These reaction temperature and time are sufficient for the hydrolysis.

The hydrolysis may be performed in the co-presence of an organic solvent such as methanol, ethanol, n-propanol, isopropanol, n-butanol, isobutanol, tertiary butanol, methyl Cellosolve and Cellosolve. Accordingly, when the reaction of the alpha,beta-dihalogenopropionitrile with ammonia or the reaction of the alpha-halogen-acrylonitrile with ammonia is carried out in a lower alcohol solvent, the alcohol may be distilled off or left in the reaction system in the subsequent hydrolysis of the reaction mixture.

The end point of the hydrolysis reaction can be rapidly and easily determined by such means as thin-layer chromatography or high-speed liquid chromatography.

The resulting product in the process of this invention can be recovered either as an alpha-halogeno-beta-alanine mineral acid salt or a free alpha-halogeno-beta-alanine depending upon the method of treatment after the hydrolysis. For example, alpha-chloro-beta-alanine hydrochloride is obtained by reacting alpha-chloroacrylonitrile with ammonia water to form alpha-chloro-beta-amino-propionitrile in the reaction mixture, subsequently subjecting the reaction mixture to hydrolysis in hydrochloric acid, concentrating the reaction mixture to dryness under reduced pressure, extracting the residue with a lower alcohol, and adding ether to the extract. Free alpha-chloro-beta-alanine is obtained if in the aforesaid process, the hydrolysis is carried out in sulfuric acid, the reaction product is neutralized with calcium hydroxide or barium hydroxide, the resulting calcium sulfate or barium sulfate is removed by filtration, and the remaining aqueous solution is concentrated to dryness under reduced pressure optionally followed by adding a lower alcohol such as methanol, ethanol, n-propanol or isopropanol.

The following Examples illustrate the present invention more specifically. All percentages in these examples are by weight.

Example 1

To 140 g of 20% hydrochloric acid was added 20.9 g of alpha-chloro-beta-aminopropionitrile, and the mixture was gradually heated with stirring and refluxed for 2 hours. Then, the reaction mixture was concentrated to dryness under reduced pressure. Hot isopropanol (250 g) was added to the residue to extract alpha-chloro-beta-alanine hydrochloride. The extract was concentrated under reduced pressure until its amount was reduced to about one-third. Addition of ether to the concentrate gave alpha-chloro-beta-alanine hydrochloride as a precipitate. The precipitate was washed with ether and dried at 40 to 50°C to afford 29.4 g (yield 92%) of alpha-chloro-beta-alanine hydrochloride as

**0 018 177**

white crystals having a melting point of 133.5 to 134.5°C.

The resulting alpha-chloro-beta-alanine hydrochloride was treated in a customary manner, for example by dissolving it in a small amount of water, neutralizing it with lithium hydroxide and then adding ethanol, thereby to isolate free alpha-chloro-beta-alanine.

The starting alpha-chloro-beta-aminopropionitrile was synthesized as follows:

243 g of concentrated ammonia water (concentration 28%) was cooled to 0°C, and with vigorous stirring in an atmosphere of nitrogen, 87.5 g of alpha-chloroacrylonitrile was added dropwise over about 2 hours. Then, the reaction was continued at 0 to 5°C for 4 hours. The excess of ammonia was removed by blowing nitrogen gas into the reaction mixture. The resulting crude alpha-chloro-beta-aminopropionitrile was extracted three times with 300 ml of 1,2-dichloroethane. The extract was dried over anhydrous sodium sulfate and distilled under reduced pressure to afford 58 g of alpha-chloro-beta-aminopropionitrile having a boiling point of 52 to 53°C/0.75 mmHg.

Example 2

To 150 g of 30% sulfuric acid was added 20.9 g of alpha-chloro-beta-aminopropionitrile, and the mixture was gradually heated with stirring. The mixture was reacted at 100 to 110°C for 6 hours. The reaction mixture was then cooled to 20°C, and calcium hydroxide in small portions was added to neutralize it to a pH of 6.5. The resulting precipitate of calcium sulfate was separated by filtration, and the residue was washed with water. The filtrate and the washing were combined, and concentrated under reduced pressure until the amount of the concentrate reached about 70 g. Ethanol was added to the concentrate. The resulting crystals were collected by filtration, washed with ethanol, and dried to afford 20.0 g (yield 80%) of free alpha-chloro-beta-alanine as white crystals having a melting point of 167 to 168°C (decomp.).

Example 3

In the reaction shown in Example 2, the reaction mixture obtained was cooled to 20°C, and 19 g of calcium hydroxide was gradually added. The resulting calcium sulfate was separated by filtration, and the residue was washed with water. The filtrate and the washing were combined, and concentrated to dryness under reduced pressure. The concentrate was extracted with 300 g of hot isopropanol. The extract was concentrated under reduced pressure until its amount was decreased to about one-third. Ether was then added. The precipitated crystals were separated by filtration, washed with ether, and dried to afford 26.7 g of white alpha-chloro-beta-alanine sulfate having a melting point of 140 to 141°C.

The elemental analysis values for $C_3H_6ClNO_2 \cdot 1/2\ H_2SO_4$ were as follows:

| | C | H | N | Cl | S |
|---|---|---|---|---|---|
| Found (%): | 20.67 | 4.20 | 7.98 | 20.72 | 9.07 |
| Calculated (%): | 20.88 | 4.09 | 8.11 | 20.57 | 9.29 |

Example 4

Thirty grams of alpha-chloro-beta-aminopropionitrile hydrochloride was added to 240 g of 20% hydrochloric acid, and the mixture was heated under reflux for 1 hour. Then, the mixture was concentrated to dryness under reduced pressure. To the residue was added 250 g of hot isopropanol to extract alpha-chloro-beta-alanine hydrochloride. The extract was concentrated under reduced pressure until its amount was decreased to about one-third. Addition of ether to the concentrate afforded a precipitate of alpha-chloro-beta-alanine hydrochloride. The precipitate was separated by filtration, washed with ether, and dried at 40°C to afford 31.6 g (yield 93%) of alpha-chloro-beta-alanine hydrochloride as white crystals having a melting point of 134 to 135°C.

The resulting alpha-chloro-beta-alanine hydrochloride was treated in a customary manner, for example by dissolving it in a small amount of water, hydrolyzing it with lithium hydroxide and then adding ethanol, thereby to isolate free alpha-chloro-beta-alanine.

Example 5

Thirty grams of alpha-chloro-beta-aminopropionitrile hydrochloride was added to 200 g of 30% sulfuric acid, and the mixture was heated at 100 to 110°C for 6 hours. Then, the reaction mixture was cooled to 20°C, and neutralized with calcium hydroxide to a pH of 6.5. The resulting precipitate of calcium sulfate was separated by filtration, and the residue was washed with water. The filtrate and the washing were combined, and concentrated under reduced pressure until the amount of the concentrate reached 80 g. Addition of ethanol to the concentrate gave a precipitate of free alpha-chloro-beta-alanine. The precipitate was filtered, washed with ethanol and dried to afford 22.6 g (yield 86%) of free alpha-chloro-beta-alanine as white crystals having a melting point of 166 to 167°C (decomp.).

6

## Example 6

In the procedure of Example 5, barium chloride was added to the reaction mixture instead of calcium hydroxide to precipitate the sulfuric acid radical in the reaction mixture as barium sulfate. The precipitate was removed, and the filtrate was concentrated to dryness under reduced pressure. The concentrate was worked up in the same way as in Example 4 to afford 30.0 g (yield 88%) of alpha-chloro-beta-alanine hydrochloride having a melting point of 133 to 135°C.

## Example 7

Thirty grams of alpha-chloro-beta-aminopropionitrile was added to 240 g of 30% sulfuric acid, and the mixture was heated at 100 to 110°C for 6 hours. After the reaction, the reaction mixture was cooled to 20°C, and 42 g of calcium hydroxide was added gradually. The resulting calcium sulfate was separated by filtration, and the residue was washed with water. The filtrate and the washing were combined, and concentrated to dryness under reduced pressure. The residue was extracted with 400 g of hot isopropanol. The extract was concentrated under reduced pressure until its amount became 120 g. Ether was added to the concentrate. The precipitated white alpha-chloro-beta-alanine sulfate was separated by filtration, washed with ether, and dried. The amount of the final product was 26.9 g (yield 80%), and its melting point was 140 to 141°C. The elemental analysis values for $C_3H_6ClNO_2 \cdot 1/2 \ H_2SO_4$ were as follows:

|                 | C     | H    | N    | Cl    | S    |
| --------------- | ----- | ---- | ---- | ----- | ---- |
| Found (%):      | 20.51 | 4.24 | 7.96 | 20.68 | 9.08 |
| Calculated (%): | 20.88 | 4.09 | 8.11 | 20.57 | 9.29 |

## Example 8

182 g of 28% ammonia water was maintained at 0°C, and with vigorous stirring in a stream of nitrogen, 87.5 g of alpha-chloroacrylonitrile was added dropwise over about 3 hours. Then, the mixture was further stirred at the same temperature for 4 hours. Nitrogen was blown into the reaction mixture to remove the unreacted ammonia. An aqueous solution consisting of 791 g of conc. hydrochloric acid and 241 g of water was added dropwise to the reaction mixture at less than 10°C, and the mixture was heated under reflux for 1 hour. Then, activated carbon was added to the reaction mixture, and it was filtered. The resulting filtrate was concentrated to dryness under reduced pressure. The residue was extracted with 800 ml of hot isopropanol. The extract was concentrated under reduced pressure until its amount was reduced to about one-third. Ether was added to the concentrate. The white crystals precipitated were separated by filtration, washed with ether, and dried at 40°C to afford 131.2 g (yield 82% based on alpha-chloro-acrylonitrile) of alpha-chloro-beta-alanine hydrochloride having a melting point of 133 to 135°C. The elemental analysis values for $C_3H_6ClNO_2 \cdot HCl$ were as follows:

|                 | C     | H    | N    | Cl    |
| --------------- | ----- | ---- | ---- | ----- |
| Found (%):      | 22.39 | 4.48 | 8.60 | 44.08 |
| Calculated (%): | 22.52 | 4.41 | 8.75 | 44.31 |

## Example 9

The procedure of Example 8 was repeated except that 132 g of alpha-bromoacrylonitrile was used instead of the alpha-chloroacrylonitrile. There was obtained 112.5 g (yield 55% based on the alpha-bromoacrylonitrile) of alpha-bromo-beta-alanine hydrochloride.

## Example 10

121 g of 28% ammonia water was maintained at 0°C, and with vigorous stirring in a nitrogen stream, 87.5 g of alpha-chloroacrylonitrile was added dropwise over about 3 hours. Then, the reaction was further carried out at the same temperature for 5 hours. Nitrogen was blown into the reaction mixture to remove the excess ammonia. Then, 900 g of 60% by weight sulfuric acid was gradually added to the reaction mixture at less than 10°C, and the mixture was heated at 100 to 105°C for 5 hours to hydrolyze the reaction product. After the hydrolysis, activated carbon was added to the reaction mixture, followed by filtration. Calcium hydroxide in small portions was added at less than 20°C to neutralize the filtrate until its pH reached 6.5. The resulting precipitate of calcium sulfate was separated by filtration, and the residue was washed with water. The filtrate and the washing were combined, and concentrated under reduced pressure until the amount of the concentrate became about 250 ml. Then, ethanol was added to the concentrate to precipitate alpha-chloro-beta-alanine. The precipitate was separated by filtration, washed with ethanol and dried under reduced pressure to afford 92.7 g (yield 75% based on alpha-chloro-acrylonitrile) of white free alpha-chloro-beta-alanine having a melting point of 166 to 167°C (decomp.). The elemental analysis values for $C_3H_6ClNO_2$ were as follows:

|  | C | H | N | Cl |
|---|---|---|---|---|
| Found (%): | 28.82 | 5.08 | 11.02 | 28.42 |
| Calculated (%): | 29.16 | 4.90 | 11.34 | 28.70 |

## Example 11

121 g of 28% ammonia water was maintained at 0°C, and with vigorous stirring in a nitrogen stream, 87.5 g of alpha-chloroacrylonitrile was added over about 2 hours. The reaction was continued further at the same temperature for 5 hours. To the reaction mixture was added gradually 900 g of 60% by weight sulfuric acid at less than 10°C, and the mixture was heated at 100 to 105°C for 5 hours to hydrolyze the reaction product. Activated carbon was added to the reaction mixture, and it was filtered. The filtrate was partially neutralized by gradually adding 220 g of calcium hydroxide at 20 to 30°C. The resulting precipitate of calcium sulfate was separated by filtration, and the residue was washed with water. The filtrate and the washing were combined, and concentrated to dryness under reduced pressure. The residue was extracted with 1000 ml of hot isopropanol. The extract was concentrated until its amount was decreased to about one-third. Addition of ether to the concentrate afforded a precipitate of white crystals. The crystals were separated by filtration, washed with a mixture of isopropanol and ether, and dried to afford 132.8 g (yield 77% based on alpha-chloroacrylonitrile) of alpha-chloro-beta-alanine sulfate having a melting point of 140 to 141°C. The elemental analysis values for $C_3H_6ClNO_2 \cdot 1/2\ H_2SO_4$ were as follows:

|  | C | H | N | Cl | S |
|---|---|---|---|---|---|
| Found (%): | 20.47 | 4.17 | 8.05 | 20.29 | 9.38 |
| Calculated (%): | 20.88 | 4.09 | 8.11 | 20.57 | 9.29 |

## Example 12

60.8 g of conc. ammonia water (concentration 28%) was maintained at 0°C, and with stirring in a gentle nitrogen stream, 24.8 g of alpha,beta-dichloropropionitrile was added dropwise over about 2 hours. Then, the reaction was further carried out at 0 to 5°C for 3 hours. Nitrogen was blown into the reaction mixture to remove the excess of ammonia. Then, 184 g of 20% hydrochloric acid was added dropwise to the reaction mixture at less than 10°C, and the mixture was heated under reflux for 2 hours. The reaction mixture was concentrated to dryness under reduced pressure, and the residue was extracted with 250 ml of hot isopropanol. The extract was concentrated. Addition of ether afforded a precipitate of white crystals. The crystals precipitated were separated by filtration, washed with ether, and dried at 40°C to afford 26.6 g (yield 83.1% based on alpha,beta-dichloropropionitrile) of alpha-chloro-beta-alanine hydrochloride having a melting point of 133 to 134.5°C. The elemental analysis values for $C_3H_6ClNO_2 \cdot HCl$ were as follows:

|  | C | H | N | Cl |
|---|---|---|---|---|
| Found (%): | 22.42 | 4.43 | 8.80 | 44.05 |
| Calculated (%): | 22.52 | 4.41 | 8.75 | 44.31 |

## Example 13

300 g of an isopropanol solution of ammonia in a concentration of 5.36% was maintained at −5°C, and with stirring in a gentle nitrogen stream, 24.8 g of alpha,beta-dichloropropionitrile was added dropwise over about 2 hours. Then, the reaction was further carried out at −5 to 0°C for 2 hours. Nitrogen was blown into the reaction mixture to remove the unreacted ammonia. Then, 184 g of 20% hydrochloric acid was added dropwise to the reaction mixture at less than 10°C. The mixture was gradually heated, and isopropanol was distilled off until the temperature of the mixture reached 106°C. The mixture was further reacted at the same temperature for 2 hours. After the hydrolysis, the reaction mixture was worked up in the same way as in Example 12 to afford 27.8 g (yield 87% based on alpha,beta-dichloropropionitrile) of alpha-chloro-beta-alanine hydrochloride having a melting point of 133.5 to 134.5°C.

## Example 14

To 250 g of methanol was added 60.8 g of conc. ammonia solution (concentration 28%), and with stirring at −5°C, 24.8 g of alpha,beta-dichloropropionitrile was added to the solution over about 2 hours. Then, the reaction was carried out for 3 hours at −5 to 0°C. Nitrogen was blown into the reaction mixture to remove the unreacted ammonia. Then, 146 g of 25% hydrochloric acid was added dropwise to the reaction mixture at less than 10°C to perform the same reaction as in Example 13.

After the hydrolysis, the reaction mixture was worked up in the same way as in Example 12 to afford 26.6 g (yield 83% based on alpha,beta-dichloropropionitrile) of alpha-chloro-beta-alanine hydrochloride having a melting point of 133 to 134.5°C.

Example 15

60.8 g of conc. ammonia water (concentration 28%) was maintained at 0°C, and with stirring in a gentle nitrogen stream, 24.8 g of alpha,beta-dichloropropionitrile was added over about 2 hours. Then, the reaction was further carried out at 0 to 5°C for 3 hours. Nitrogen was blown into the reaction mixture to remove the unreacted ammonia. Then, 200 g of 60% sulfuric acid was added dropwise to the reaction mixture at less than 10°C. The mixture was gradually heated, and hydrolyzed at 100 to 105°C for 5 hours. After the hydrolysis, the reaction mixture was neutralized to a pH of 6.5 by adding calcium hydroxide in small portions at less than 20°C. The resulting precipitate of calcium sulfate was separated by filtration, and the residue was washed with water. The filtrate and the washing were combined, and concentrated under reduced pressure until the amount of the concentrate became about 100 ml. Ethanol was then added to the concentrate to precipitate alpha-chloro-beta-alanine. The precipitate was separated by filtration, washed with ethanol and dried under reduced pressure to afford 18.5 g (yield 75% based on alpha,beta-dichloropropionitrile) of white free alpha-chloro-beta-alanine having a melting point of 166 to 167.5°C (decomp.). The elemental analysis values for $C_3H_6ClNO_2$ were as follows:

|  | C | H | N | Cl |
|---|---|---|---|---|
| Found (%): | 28.85 | 4.78 | 11.12 | 28.97 |
| Calculated (%): | 29.16 | 4.90 | 11.34 | 28.70 |

**Claims**

1. A process for producing an alpha-halogeno-beta-alanine or its mineral acid salt, which comprises hydrolyzing an alpha-halogeno-beta-aminopropionitrile or its mineral acid salt as a starting material with a mineral acid.

2. The process of claim 1 wherein said alpha-halogeno-beta-aminopropionitrile is alpha-chloro-beta-amino-propionitrile or alpha-bromo-beta-aminopropionitrile, or wherein said alpha-halogeno-beta-aminopropionitrile mineral acid salt is alpha-chloro-beta-aminopropionitrile hydrochloride, alpha-bromo-beta-aminopropionitrile hydrochloride, alpha-chloro-beta-aminopropionitrile sulfate or alpha-bromo-beta-aminopropionitrile sulfate.

3. The process of claim 1 or claim 2 wherein the concentration of the mineral acid is 3 to 80% by weight.

4. The process of claim 1, claim 2 or claim 3 wherein said mineral acid used for the hydrolysis is hydrochloric acid preferably having a concentration of 3 to 35% by weight, sulfuric acid, preferably having a concentration of 3 to 60% by weight, nitric acid or phosphoric acid.

5. The process of any one of the preceding claims wherein said hydrolysis is carried out at a temperature of from 50 to 150°C.

6. The process of any one of the preceding claims wherein said starting material is the reaction mixture containing an alpha-halogeno-beta-aminopropionitrile and obtained by reacting an alpha,beta-dihalogenopropionitrile, preferably alpha-beta-dichloropropionitrile or alpha,beta-dibromopropionitrile, with ammonia in water and/or an organic solvent.

7. The process of claim 6 wherein said organic solvent is a lower alcohol.

8. The process of claim 6 or claim 7 wherein the reaction temperature is —40°C to 30°C.

9. The process of any one of claims 1 to 5 wherein said starting material is the reaction mixture containing an alpha-halogeno-beta-aminopropionitrile and obtained by the addition reaction of an alpha-halogenoacrylonitrile with ammonia.

10. The process of claim 9 wherein said alpha-halogenoacrylonitrile is alpha-chloroacrylonitrile or alpha-bromoacrylonitrile.

**Revendications**

1. Procédé de production d'une alpha-halogéno-béta-alanine ou son sel d'acide minéral, qui consiste à hydrolyser un alpha-halogéno-béta-aminopropionitrile ou son sel d'acide minéral en tant que matière première, avec un acide minéral.

2. Procédé selon la revendication 1 où l'alpha-halogéno-béta-aminopropionitrile est de l'alpha-chloro-béta-aminopropionitrile ou de l'alpha-bromo-béta-aminopropionitrile ou bien où ledit sel d'acide minéral d'alpha-halogéno-béta-aminopropionitrile est le chlorhydrate d'alpha-chloro-béta-aminopropionitrile, le chlorhydrate d'alpha-bromo-béta-aminopropionitrile, le sulfate d'alpha-chloro-béta-aminopropionitrile ou le sulfate d'alpha-bromo-béta-aminopropionitrile.

3. Procédé selon la revendication 1 ou la revendication 2 où la concentration de l'acide minéral est de 3 à 80% en poids.

4. Procédé selon la revendication 1, la revendication 2 ou la revendication 3 où l'acide minéral utilisé pour l'hydrolyse est de l'acide chlorhydrique ayant de préférence une concentration de 3 à 35% en poids, de l'acide sulfurique ayant de préférence une concentration de 3 à 60% en poids, de l'acide nitrique ou de l'acide phosphorique.

5. Procédé selon l'une quelconque des revendications précédentes où l'hydrolyse est effectuée à une température de 50 à 150°C.

6. Procédé selon l'une quelconque des revendications précédentes où la matière première est le mélange réactionnel contenant un alpha-halogéno-béta-aminopropionitrile et obtenu par réaction d'un alpha,béta-dihalogénopropionitrile, de préférence de l'alpha-béta-dichloropropionitrile ou de l'alpha,béta-dibromopropionitrile avec de l'ammoniac dans l'eau et/ou un solvant organique.

7. Procédé selon la revendication 6, où le solvant organique est un alcool inférieur.

8. Procédé selon la revendication 6 ou la revendication 7 où la température de la réaction est de —40 à 30°C.

9. Procédé selon l'une quelconque des revendications 1 à 5, où la matière première est le mélange réactionnel contenant un alpha-halogéno-béta-aminopropionitrile et obtenu par la réaction d'addition d'un alpha-halogéno acrylonitrile avec de l'ammoniac.

10. Procédé selon la revendication 9, où l'alpha-halogénoacrylonitrile est de l'alpha-chloroacrylonitrile ou de l'alpha-bromoacrylonitrile.

**Patentansprüche**

1. Verfahren zur Herstellung eines $\alpha$-Halogen-$\beta$-alanins oder dessen Mineralsäuresalzes, bei dem ein $\alpha$-Halogen-$\beta$-aminopropionitril oder dessen Mineralsäuresalz als Ausgangsmaterial mit einer Mineralsäure hydrolysiert wird.

2. Verfahren nach Anspruch 1, bei dem das $\alpha$-Halogen-$\beta$-aminopropionitril $\alpha$-Chlor-$\beta$-aminopropionitril oder $\alpha$-Brom-$\beta$-aminopropionitril ist oder bei dem das $\alpha$-Halogen-$\beta$-aminopropionitril-Mineralsäuresalz $\alpha$-Chlor-$\beta$-aminopropionitril-Hydrochlorid, $\alpha$-Brom-$\beta$-aminopropionitril-Hydrochlorid, $\alpha$-Chlor-$\beta$-aminopropionitril-Sulfat oder $\alpha$-Brom-$\beta$-aminopropionitril-Sulfat ist.

3. Verfahren nach Anspruch 1 oder 2, bei dem die Konzentration der Mineralsäure 3 bis 80 Gew.—% ist.

4. Verfahren nach Anspruch 1, 2 oder 3, bei dem die zur Hydrolyse verwendete Mineralsäure Salzsäure, vorzugsweise mit einer Konzentration von 3 bis 35 Gew.—%, Schwefelsäure, vorzugsweise einer Konzentration von 3 bis 60 Gew.—%, Salpetersäure oder Phosphorsäure ist.

5. Verfahren nach einem der vorhergehenden Ansprüche, bei dem die Hydrolyse bei einer Temperatur von 50 bis 150°C erfolgt.

6. Verfahren nach einem der vorhergehenden Ansprüche, bei dem das Ausgangsmaterial das ein $\alpha$-Halogen-$\beta$-aminopropionitril enthaltende Reaktionsgemisch, erhalten durch Umsetzen eines $\alpha,\beta$-Dihalogenpropionitrils, vorzugsweise $\alpha,\beta$-Dichlorpropionitrils oder $\alpha,\beta$-Dibrompropionitrils, mit Ammoniak in Wasser und/oder einem organischen Lösungsmittel, ist.

7. Verfahren nach Anspruch 6, bei dem das organische Lösungsmittel ein niederer Alkohol ist.

8. Verfahren nach Anspruch 6 oder 7, bei dem die Reaktionstemperatur —40°C bis 30°C ist.

9. Verfahren nach einem der Ansprüche 1 bis 5, bei dem das Ausgangsmaterial das ein $\alpha$-Halogen-$\beta$-aminopropionitril enthaltende Reaktionsgemisch, erhalten durch Additionsreaktion eines $\alpha$-Halogenacrylnitrils mit Ammoniak, ist.

10. Verfahren nach Anspruch 9, bei dem das $\alpha$-Halogen-acrylnitril $\alpha$-Cloracrylnitril oder $\alpha$-Bromacrylnitril ist.